# FASCICULE DE BREVET EUROPEEN

(11) **EP 1 032 376 B1**
(45) Date de publication et mention de la délivrance du brevet: **02.05.2002**
(21) Numéro de dépôt: 98955701.2
(22) Date de dépôt: 19.11.1998
(51) Int. Cl.: A61K 31/165

(54) **UTILISATION DE DERIVES DE BENZHYDRYL SULFINYLE POUR TRAITER LA SOMNOLENCE D'ORIGINE MEDICAMENTEUSE**
VERWENDUNG VON BENZHYDRYLSULFINYLDERIVATEN ZUR BEHANDLUNG DER SCHLÄFIGKEIT MEDIKAMENTÖSEN URSPRUNGS
USE OF SULPHINYL BENZHYDRYL DERIVATIVES FOR TREATING DRUG-INDUCED SLEEPINESS

(30) Priorité: 19.11.1997 FR 9714519
(43) Date de publication de la demande: 06.09.2000
(73) Titulaire: Institut Curie, 75248 Paris Cedex 05 (FR)
(72) Inventeur: ESTEVE, Marc, F-94100 Saint-Maur des Fosses (FR); GERTNER, Jacques, F-75014 Paris (FR)
(74) Mandataire: Peaucelle, Chantal
(86) Numéro de dépôt international: FR9802478
(87) Numéro de publication internationale: WO9925329

(56) Documents cités:
- GB-A- 1 584 462
- ROTH T. ET AL: "Etiologies and sequelae of excessive daytime sleepiness" CLINICAL THERAPEUTICS, 1996, 18/4 (562-576), XP002075153 USA
- RAMBERT F A ET AL: "PSYCHOPHARMACOLOGICAL STUDIES WITH ADRAFINIL A UNIQUE BEHAVIORAL PROFILE IN MICE" J PHARMACOL (PARIS), 17 (1). 1986. 37-52., XP002075154
- "British National Formulary" 1986 , THE PHARMACEUTICAL PRESS , LONDON XP002094890 11 voir page 156 voir page 166

## Description

L'invention concerne une nouvelle utilisation en thérapeutique de dérivés de benzhydryl sulfinyle.

Elle concerne, plus spécialement, l'utilisation de tels dérivés dans des situations de troubles de la vigilance liés à un traitement anti-douleur tel qu'appliqué dans des pathologies lourdes comme le cancer, ou pour les séquelles douloureuses d'affections graves.

40% environ des patients cancéreux ont en effet au cours de l'évolution de leur maladie à faire face à la douleur.

Celle-ci survient soit du fait de la progression défavorable du cancer, soit du fait de séquelles des différents traitements entrepris.

L'Organisation Mondiale pour la Santé a établi, il y a plusieurs années, un certain nombre de principes pour la prise en charge de la douleur en cancérologie. Elle a notamment rappelé la place importante que devait tenir la morphine dans ce traitement.

Grâce à cette impulsion, et malgré les préjugés culturels tournant autour de la morphine, ce produit est maintenant prescrit et accepté de plus en plus facilement.

Si sa grande efficacité sur le plan antalgique n'est plus à démontrer, on ne peut pas passer sous silence ses effets secondaires, en particulier la somnolence qu'elle provoque. Les différentes études qui l'ont évaluée rapportent qu'elle gène 30 à 50% des patients sous morphine lorsque celle-ci est prise dans le cadre d'un traitement chronique du cancer.

Les démarches de recherche actuelles dans le traitement de la douleur sont en majorité orientées vers la perspective de voir diminuer ces effets secondaires, et tout particulièrement la somnolence.

Les produits dont dispose le clinicien à ce jour sont essentiellement des dérivés amphétaminiques. Cependant de tels dérivés sont à l'origine d'inconvénients majeurs, liés à leurs effets cardiovasculaires et neuropsychiques indésirables d'une part, et de dépendance lors de l'usage à long terme, d'autre part.

En recherchant une solution à ce problème, les inventeurs se sont orientés vers l'évaluation des effets, dans un contexte de traitement morphinique, de composés connus pour leur pouvoir éveillant et leur capacité à stimuler la vigilance.

Ils ont pu ainsi vérifier que, de manière inattendue, de tels composés antagonisaient sélectivement l'effet hypnogène de la morphine, sans affecter son activité antalgique, et ce sans créer d'autres inconvénients.

Il a également été vérifié que ces effets antagonistes sélectifs s'exerçaient aussi vis-à-vis des états de somnolence induits par les médicaments classiquement administrés avec la morphine, tels que les antalgiques, anti-dépresseurs et anxyolytiques.

L'invention vise donc l'utilisation, pour la fabrication de médicaments, de composés capables d'exercer un effet éveillant dans des situations de troubles de la vigilance liés à un traitement morphinique (cette expression englobant l'utilisation de morphine ou dérivés de celle-ci avec le cas échéant les médicaments couramment utilisés dans ce type de contexte).

Elle vise également de nouvelles présentations pharmaceutiques permettant d'obtenir conjointement l'ensemble des effets recherchés.

Conformément à l'invention, on utilise, pour la fabrication desdits médicaments, des composés de benzhydryl sulfinyle répondant à la formule générale (I), 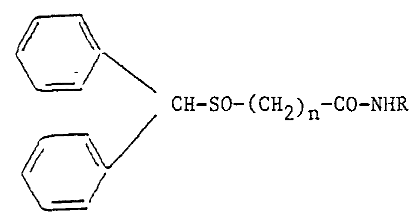 dans laquelle,
- chacun des cycles peut être substitué par un ou plusieurs groupes F, Cl, Br, CF₃, NO₂, NH₂, alkyle en C₁-C₄, alkoxy en C₁-C₄, méthylènedioxy ;
- R représente un groupe hydroxyle, un atome d'hydrogène, un groupe alkyle en C₁-C₄, un groupe hydroxyalkyle en C₁-C₄,
- n est un entier égal à 1,2 ou 3 ; et leurs sels d'addition lorsque R comporte un reste basique.

De manière préférée, R représente un groupe -OH ou un atome d'hydrogène.

L'invention vise en particulier l'utilisation de l'acide benzhydryl sulfinyl-acétohydroxamique de formule (II), 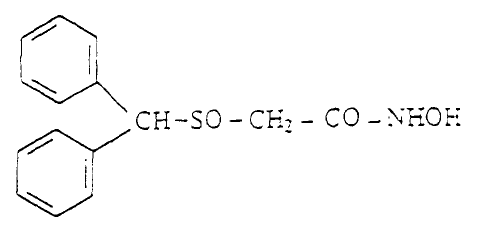 désigné par adrafinil selon la dénomination comme internationale et commercialisé sous la marque Olmifon®.

Elle vise tout spécialement l'utilisation de son métabolite, à savoir le benzhydryl sulfinyl acétamide de formule (III), 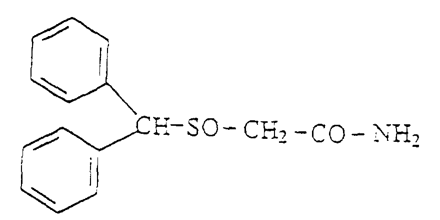 répondant à la dénomination commune internationale de modafinil et commercialisé sous la marque Modiodal®.

Les composés utilisés selon l'invention sont connus pour leur activité sélective de stimulation de l'éveil et de la vigilance et sont largement utilisés pour le traitement de la narcolepsie et de l'hypersomnie idiopathique.

De manière surprenante, utilisés dans le contexte d'un traitement morphinique, associé le cas échéant à l'administration d'anti-dépresseurs et/ou anxiolytiques et/ou antalgiques, ils permettent de réduire considérablement l'état de somnolence, évalué selon l'échelle Epworth, tout en assurant le maintien des propriétés antalgiques de la morphine et celles des médicaments anti-dépresseurs, anxiolytiques ou antalgiques. Le patient se trouve ainsi rétabli dans une vie relationelle satisfaisante.

Selon un mode de réalisation avantageux de l'invention, lesdits médicaments renferment au moins un composé de formule (I), à raison de 50 à 600 mg de préférence de 100 à 300 mg.

Lors de l'élaboration des médicaments, les principes actifs sont mélangés avec les véhicules pharmaceutiquement acceptables pour le mode d'administration choisi.

Ainsi pour une administration par voie orale, les médicaments sont préparés sous forme de gélules, comprimés, dragées, capsules, et analogues.

Pour l'administration par voie injectable, les médicaments se présentent sous forme de solutions dans des ampoules injectables.

On peut également avoir recours à une administration par voie transcutanée, sous forme de patch.

Comme exposé dans les exemples, des résultats très favorables ont été obtenus en clinique avec des administrations de 200 à 400 mg environ par jour de Modiodal®, en 1 ou 2 prises, et de 1200 à 1800 mg environ par jour d'Olmifon® en 2 prises.

Selon un autre aspect mettant à profit les effets résultant de l'administration conjointe d'antalgiques, et/ou d'anti-dépresseurs et/ou anxiolytiques d'une part, et de produits stimulants la vigilance d'autre part, l'invention fournit une présentation pharmaceutique caractérisée en ce qu'elle renferme respectivement les deux types de médicaments, avec une notice d'information appropriée.

Dans cette présentation, les médicaments sont sous les formes galéniques appropriées pour la voie d'administration choisie.

Afin d'illustrer l'invention, sans toutefois en limiter sa portée, on rapporte ci-après les résultats d'observations effectuées sur des patients, de manière confidentielle, dans le cadre d'une hospitalisation. Le consentement des patients a été obtenu après leur avoir expliqué les modalités compassionelles de la prescription.

### CAS N°1

Madame M. née, en 1937, est atteinte d'un cancer du sein droit depuis 1991. Après avoir subi un traitement local chirurgical et radiothérapeutique, elle présente en 1993 les premières douleurs révélatrices d'une diffusion. métastique osseuse.

Un traitement chimiothérapique est immédiatement instauré.

Ces premières difficultés douloureuses la conduisent en consultation sur la douleur, où est initié rapidement un traitement morphinique. Celui-ci la soulage, mais occasionne un degré de somnolence important allant jusqu'à limiter, voire arrêter, sa prescription à la demande de la patiente. Entre 1994 et 1997 Madame M. est régulièrement suivie à la consultation de la douleur.

Le traitement morphinique est repris à plusieurs reprises du fait de la survenue d'épisodes douloureux en rapport avec l'éclosion de nouveaux foyers métastatiques osseux, puis arrêté lorsque l'efficacité du traitement radiothérapique, alors institué, est obtenu.

A partir de janvier 1996, le traitement morphinique ne pourra plus être interrompu. Son efficacité est modérée du fait de la limitation de sa progression en posologies eu égard à la somnolence qu'il occasionne.

Devant la progression des phénomènes douloureux, fin octobre 1997, la patiente est hospitalisée. A ce moment, la posologie du Moscontin® est à 100 mg deux fois par 24 heures, son score EVA est à 60, sa somnolence évaluée sur l'échelle d'Epworth est à 20. Une augmentation du Moscontin® à 160 mg deux fois par 24 heures est effectuée. Après stabilisation du niveau antalgique à un score EVA de 30, un traitement par Modiodal®, initialement à la posologie de 100 mg puis rapidement à 200 mg est instauré. Cette posologie permet de voir son score d'Epworth chuter au-dessous de 10. Cette qualité d'éveil lui a permis une reprise des relations familiales de meilleure qualité. L'efficacité de l'analgésie et son meilleur niveau de vigilance lui ont rendu possibles des promenades accompagnées. Il est a noter aussi chez cette patiente une reprise alimentaire se traduisant par une prise pondérale de 2 kgs en 10 jours.

### CAS N°2

Madame B. née, en 1952, présente depuis 1987 un cancer du sein.

Son évolution s'est faite sur le mode métastatique aux niveaux pulmonaire, hépatique et osseux.

En septembre 1997, apparaissent des douleurs très invalidantes costales droites qui la conduisent à la consultation de la douleur. Conjointement à des flashs de radiothérapie, est débuté un traitement antalgique qui associe dextropropoxyphène, paracétamol et clonazépam. La patiente a déjà au cours de sa maladie pris de la morphine orale et redoute ce produit du fait de ses effets hypnotiques. Elle souhaite continuer d'exercer son métier. Devant la persistance des phénomènes douloureux le passage à la morphine orale s'avère obligatoire. Le traitement se fait par Skénan®, à la dose de 30 mg, deux fois par 24 heures. On revoie au bout de 48 heures la patiente. Elle évalue sa douleur à 30 sur l'échelle EVA, mais se sent très somnolente. Elle ne souhaite pas augmenter la posologie morphinique. Sept jours plus tard la patiente consulte à nouveau, la douleur est côtée à 60 et son état de somnolence est important, côté à 16 sur l'échelle d'Epworth. L'hospitalisation dans le but d'équilibrer douleur et somnolence est acceptée.

Les doses de Skénan® sont doublées d'emblée et on leur associe immédiatement 200 mg de Modiadal®. Le résultat est rapidement favorable tant sur le plan antalgique (EVA 20) qu'au niveau de la vigilance (cotation inférieure à 10). A la sortie de l'hôpital Madame B. a pu reprendre ses activités professionnelles.

### CAS N°3

Monsieur L., né en 1922, est traité depuis 1994 pour un cancer de la prostate. En juillet 1997 une localisation vertébrale cervicale se révèle par des signes de compression médullaire. Une laminectomie est pratiquée dans un premier temps suivie d'une radiothérapie. Ces traitements vont permettre de stabiliser les lésions neurologiques. Cependant Monsieur L. va conserver des douleurs cervicales qui amènent à débuter un traitement antalgique par morphine orale à libération prolongée. Ce traitement, consistant à administrer du Moscontin®, à raison de 30 mg, deux fois par 24 heures, sera mal supporté tant sur le plan digestif (nausées) que cognitif (désorientation importante). Une première hospitalisation est proposée. Elle permettra la mise en route d'un traitement par morphine sous-cutanée, en continu, à la seringue électrique associé à du Clonazépam par voie orale. Le patient sort de l'hôpital, la côtation de sa douleur sur l'échelle EVA est à 30. Il persiste cependant une somnolence importante.

Sur le plan vertébral, les signes de compression neurologique vont réapparaître progressivement. Les conditions de soin devenant difficiles à domicile, Monsieur L. est réhospitalisé mi-octobre. A l'arrivée en hospitalisation, sa douleur cervicale est toujours contrôlée par une dose modérée de morphine (40 mg) par voie sous-cutanée. Il présente cependant un état de somnolence majeur empêchant tout dialogue suivi avec sa famille. (20 sur l'échelle d'Epworth). Un traitement par Modiodal® 100 mg, puis 200 mg, va lui permettre de recouvrir un état de vigilance nettement supérieur côté à 12.

### CAS N°4

Madame Q., née en 1929, Oa été énuclée de l'oeil gauche en 1995 pour un mélanome de la choroïde au-dessus de toute ressource thérapeutique conservatrice. En 1997, apparaissent des métastases hépatiques pour lesquelles un traitement chimiothérapique est effectué. Au mois d'octobre 1997, elle consulte pour des douleurs vertébrales côtées 60 à l'EVA. Un traitement morphinique par voie orale Moscontin®, 100 mg x 2) va permettre de stabiliser ces phénomènes doulouréux (EVA = 40), mais au prix d'un état de somnolence invalidant. Une hospitalisation dans le but de régler le problème de la somnolence est acceptée par la patiente. Un traitement par modafinil va permettre, à la dose de 300 mg, progressivement obtenue, de voir passer l'index de somnolence de 23 à 11.

### CAS N°5

Madame B., née en 1911, est suivie pour un cancer du sein. Son évolution métastatique s'est faîte au niveau osseux. Elle présente une métastase sus cotyloidienne très douloureuse lui empêchant toute mobilité de la hanche. Une radiothérapie n'a pu régler le problème algique. Un traitement morphinique va être instituer à domicile, il va très rapidement entraîner une somnolence forte, rendant les soins à la maison impossibles. L'antalgie n'est pas correctement obtenue (EVA = 40). L'hospitalisation va permettre de régler le problème antalgique en utilisant la morphine par voie sous-cutanée à raison de 40 mg (équivalent-dose supérieur à la dose qu'elle prenait par voie orale) ainsi que la somnolence, par une dose de 200 mg de modafinil par jour (l'index de somnolence va passer de 22 à 15).

Ces cas pilotes montrent l'efficacité du modafinil sur la somnolence morphinique, voire sur celle associée au clonazépam.

L'invention fournit ainsi les moyens d'activer les structures d'éveil d'un patient sous traitement morphinique entraînant un état de somnolence, sans effet sur le système périphérique. L'invention permet en particulier un retour au moins partiel, voire total, à une condition physique normale pour le patient atteint d'un cancer ou souffrant de séquelles douloureuses d'affections graves. Elle permet également d'augmenter la tolérance à la morphine.

## Revendications

1. Utilisation, pour la fabrication de médicaments à effet d'éveil dans des situations de troubles de la vigilance liés à un traitement morphinique, des composés de benzhydryl sulfinyle répondant à la formule générale (I), 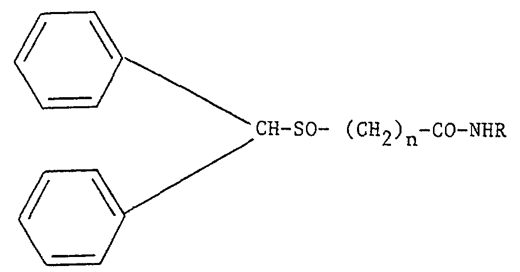 dans laquelle,
- chacun des cycles peut être substitué par un ou plusieurs groupes F, Cl, Br, CF₃, NO₂, NH₂, alkyle en C₁-C₄, alkoxy en C₁-C₄, méthylènedioxy ;
- R représente un groupe hydroxyle, un atome d'hydrogène, un groupe alkyle en C₁-C₄, un groupe hydroxyalkyle en C₁-C₄,
- n est un entier égal à 1,2 ou 3 ; et leurs sels d'addition lorsque R comporte un reste basique.

2. Utilisation selon la revendication 1, **caractérisée en ce que** R représente un groupe -OH ou un atome d'hydrogène.

3. Utilisation selon la revendication 2, **caractérisée en ce que** le composé de benzhydryl sulfinyle est l'acide benzhydryl sulfinyl-acétohydroxamique de formule (II),

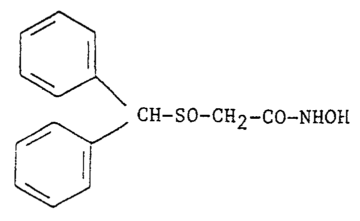

4. Utilisation selon la revendication 2, **caractérisée en ce que** le composé de benzhydryl sulfinyle est le benzhydryl sulfinyl-acétamide de formule (III),

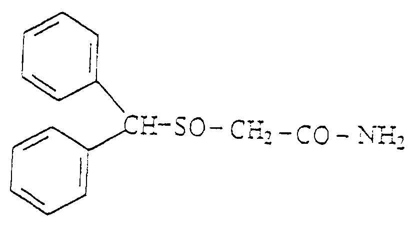

5. Utilisation selon l'une quelconque des revendications 1 à 4, **caractérisée en ce que** lesdits médicaments renferment les composés de benzhydryl sulfinyle à raison de 50 à 600 mg, de préférence de 100 à 300 mg.

6. Utilisation selon la revendication 5, **caractérisée en ce que** les médicaments sont préparés pour une administration par voie orale, sous forme de gélules, comprimés, dragées ou capsules.

7. Utilisation selon la revendication 5, **caractérisée en ce que** les médicaments sont préparés pour une administration par voie injectable, sous forme de solutions dans des ampoules injectables.

8. Utilisation selon la revendication 5, **caractérisée en ce que** les médicaments sont préparés pour une administration par voie transcutanée, sous forme de patch.

9. Produits contenant de la morphine et au moins un dérivé de benzhydryl sulfinyle selon l'une des revendications 1 à 4, comme produit de combinaison pour une utilisation simultanée, ou étalée dans le temps dans un traitement anti-douleur.

## Claims

1. Use, for the manufacture of medicaments having an awakening effect in situations of disorders in vigilance associated with a morphine treatment, of benzhydryl sulphinyl compounds corresponding to the general formula (I) 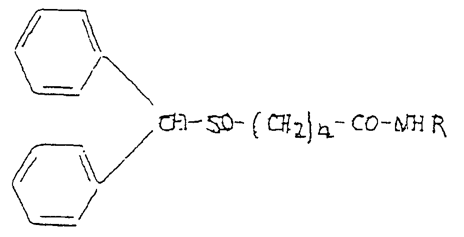 in which
- each of the rings can be substituted by one or more F, Cl, Br, CF₃, NO₂, NH₂, C₁-C₄-alkyl, C₁-C₄-alkoxy or methylenedioxy groups;
- R represents a hydroxyl group, a hydrogen atom, a C₁-C₄-alkyl group or a C₁-C₄-hydroxyalkyl group;
- n is an integer equal to 1, 2 or 3; and their addition salts when R contains a basic radical.

2. Use according to claim 1, **characterized in that** R represents an -OH group or a hydrogen atom.

3. Use according to claim 2, **characterized in that** the benzhydryl sulphinyl compound is benzhydryl sulphinyl-acetohydroxamic acid of formula (II)

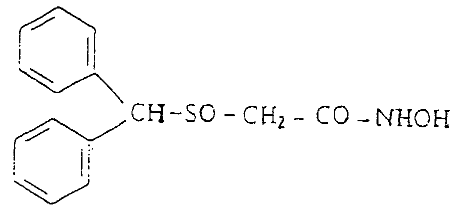

4. Use according to claim 2, **characterized in that** the benzhydryl sulphinyl compound is benzhydryl sulphinyl-acetamide of formula (III)

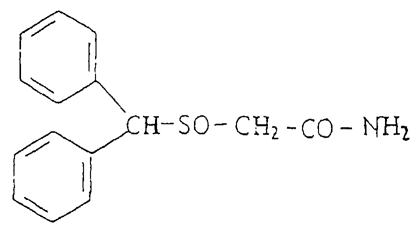

5. Use according to any one of claims 1 to 4, **characterized in that** the said medicaments comprise the benzhydryl sulphinyl compounds in an amount of 50 to 600 mg, preferably 100 to 300 mg.

6. Use according to claim 5, **characterized in that** the medicaments are prepared for administration by oral route in the form of gelatine capsules, tablets, coated tablets or capsules.

7. Use according to claim 5, **characterized in that** the medicaments are prepared for administration by injectable route in the form of solutions in injectable ampoules.

8. Use accor ding to claim 5, **characterized in that** the medicaments are prepared for administration by transcutaneous route in the form of a patch.

9. Products containing morphine and at least one benzhydryl sulphinyl derivative according to one of claims 1 to 4, as a combination product for simultaneous use or a use spread over a period of time in an anti-pain treatment.

## Patentansprüche

1. Verwendung von Benzhydrylsulfinylverbindungen mit der allgemeinen Formel (I) 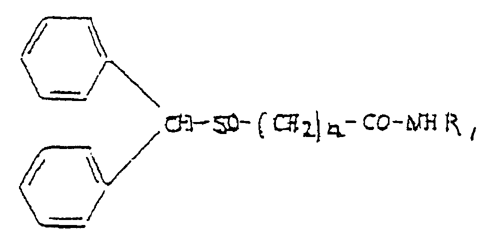 in welcher
- jeder Cyclus durch eine oder mehrere F-, Cl-, Br-, CF₃-, NO₂-, NH₂-, C₁- bis C₄-Alkyl-, C₁- bis C₄-Alkoxy- und Methylendioxy-Gruppen substituiert sein kann,
- R eine Hydroxylgruppe, ein Wasserstoffatom, eine C₁- bis C₄-Alkylgruppe und eine C₁- bis C₄-Hydroxyalkylgruppe und
- n eine ganze Zahl von 1, 2 oder 3 bedeutet,
und ihrer Additionssalze, wenn R einen basischen Rest umfasst, zur Herstellung von Arzneimitteln mit muntermachender Wirkung bei mit einer Morphinbehandlung verbundenen Aufmerksamkeitsstörungen.

2. Verwendung nach Anspruch 1, **dadurch gekennzeichnet, dass** R eine OH-Gruppe oder ein Wasserstoffatom bedeutet.

3. Verwendung nach Anspruch 2, **dadurch gekennzeichnet, dass** die Benzhydrylsulfinylverbindung das Benzhydrylsulfinylacetohydroxamid ist mit der Formel (II)

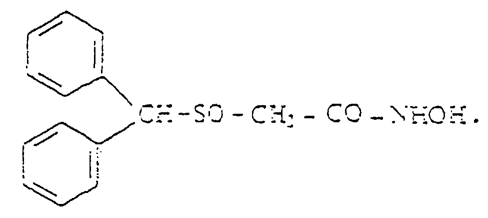

4. Verwendung nach Anspruch 2, **dadurch gekennzeichnet, dass** die Benzhydrylsulfinylverbindung das Benzhydrylsulfinylacetamid ist mit der Formel (III)

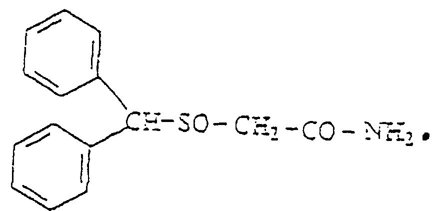

5. Verwendung nach einem der Ansprüche 1 bis 4, **dadurch gekennzeichnet, dass** die Arzneimittel die Benzhydrylsulfinylverbindungen in einer Menge von 50 bis 600 mg und vorzugsweise von 100 bis 300 mg enthalten.

6. Verwendung nach Anspruch 5, **dadurch gekennzeichnet, dass** die Arzneimittel für eine orale Verabreichung in Form von Gelatinekapseln, Tabletten, Dragees oder Kapseln hergestellt sind.

7. Verwendung nach Anspruch 5, **dadurch gekennzeichnet, dass** die Arzneimittel für eine injizierbare Verabreichung in Form von Lösungen in Injektionsampullen hergestellt sind.

8. Verwendung nach Anspruch 5, **dadurch gekennzeichnet, dass** die Arzneimittel für eine transkutane Verabreichung in Pflasterform hergestellt werden.

9. Erzeugnisse, die Morphin und mindestens ein Benzhydrylsulfinylderivat nach einem der Ansprüche 1 bis 4 enthalten, als Kombinationserzeugnis für eine Verabreichung, die bei einer Schmerztherapie auf einmal oder über die Zeit verteilt erfolgt.
